# EUROPÄISCHE PATENTANMELDUNG

(11) **EP 1 512 757 A2**
(43) Veröffentlichungstag der Anmeldung: **09.03.2005**
(21) Anmeldenummer: 04090345.2
(22) Anmeldetag: 06.09.2004
(51) Int. Cl.: C12Q 1/68, B01L 7/00

(54) **Verfahren zur Validierung von Thermocyclern**

(30) Priorität: 05.09.2003 DE 10341874
(71) Anmelder: Congen Biotechnologie GmbH, 13125 Berlin (DE)
(72) Erfinder: Kuhn, Mathias, Dr., 10437 Berlin (DE); Mergemeier, Steffen, Dr., 10243 Berlin (DE)
(74) Vertreter: Ziebig, Marlene, Dr. Dipl.-Chem.

(57) **Zusammenfassung**

Die Erfindung betrifft ein Verfahren zur Validierung oder Referenzierung von Thermocyclern, insbesondere in PCR-Geräten, wobei das Verfahren mindestens zwei Reaktionen umfasst, bei der die Annealingtemperatur und die Denaturierungstemperatur beispielsweise einer Sequenzierungsreaktionen oder Amplifikationsreaktionen überprüft werden, die Erfindung betrifft auch einen Kit und seine Verwendung zur Validierung von Thermocyclern.

## Beschreibung

Die Erfindung betrifft ein Verfahren zur Validierung oder Referenzierung von Thermocyclern, insbesondere in PCR-Geräten, wobei das Verfahren mindestens zwei Reaktionen umfasst, bei der die Annealingtemperatur und die Denaturierungstemperatur beispielsweise einer Sequenzierungsreaktionen oder Amplifikationsreaktionen überprüft werden, die Erfindung betrifft auch einen Kit und seine Verwendung zur Validierung von Thermocyclern.

Die Polymerase-Kettenreaktion (PCR) wurde in den frühen 1980er Jahren von Kary Banks Mullis entwickelt. Seine Idee war es, ein Verfahren zu entwickeln, das DNA durch wiederholte Verdoppelung in mehreren Zyklen mit Hilfe eines Enzyms namens DNA-Polymerase künstlich vervielfältigt. DNA-Polymerase kommt in allen Lebewesen vor, sie verdoppelt die DNA vor der Zellteilung. Sie bindet an einen einzelnen DNA-Strang und erzeugt einen dazu komplementären Strang. Bereits in Mullis' ursprünglichem PCR-Versuch wurde das Enzym in vitro (in einer kontrollierten, künstlichen Umgebung) verwendet. Die doppelsträngige DNA wurde durch Erhitzen auf 96°C in zwei Einzelstränge aufgeteilt. Bei dieser Temperatur wurde die DNA-Polymerase zerstört und musste daher nach jedem Erhitzen erneuert werden. Mullis' ursprüngliches Verfahren war sehr ineffizient, da es viel Zeit, große Mengen DNA-Polymerase und ständige Aufmerksamkeit erforderte. Später wurde der PCR-Prozess verbessert, indem man die DNA-Polymerase von thermophilen Bakterien verwendete, die bei über 110°C in Geysiren leben. Die DNA-Polymerase dieser Lebewesen ist und wurde daher beim Erhitzen während der PCR-Zyklen nicht zerstört. Da nicht mehr ständig neue DNA-Polymerase hinzugefügt werden musste, konnte der Vervielfältigungs-Vorgang erheblich vereinfacht und automatisiert werden. Eine der ersten thermostabilen DNA-Polymerasen wurde aus *Thermophilus aquaticus* gewonnen und Taq genannt. Taq-Polymerase erfährt gegenwärtig breite Anwendung. Ein Nachteil der Taq-Polymerase liegt darin, dass sie manchmal Fehler beim Kopieren der DNA produziert, was zu Mutationen (Fehlern) in der DNA-Sequenz führt. Polymerasen wie Pwo oder Pfu, die aus Archaea gewonnen werden, haben einen Korrektur-Mechanismus, der die Anzahl der Mutationen in der kopierten DNA erheblich senkt. Aber zahlreiche chemische Reaktionen, wie beispielsweise Sequenzierungsreaktionen oder Amplifikationsreaktionen, erreichen ihre optimale Effizienz und Effektivität erst bei der Einhaltung eines bestimmten definierten Temperaturregimes.

Mit der Etablierung biochemischer Verfahren in den Laborroutinen der Grundlagenforschung und medizinischer Klinik war es daher üblich geworden, zahlreiche in-vitro-Reaktionen beispielsweise in einem Wasserbad durchzuführen. Wenn mehrere Reaktionsansätze, beispielsweise in Reagenzgläsern oder Kunststoffgefäßen, wie beispielsweise Tubes, zeitgleich in das identische Wasserbad gesetzt werden, ist gewährleistet, dass alle Reaktionsansätze mit der gleichen Umgebungstemperatur in Kontakt gebracht werden.

So nutzte beispielsweise Kary Mullis bei der Erfindung der PCR (Polymerase Chain Reaction) noch verschiedene Wasserbäder, in denen er seine jeweiligen Reaktionsansätze positionierte. Mit der diagnostischen Anwendung von Laborroutinen und der Zunahme von Paralleluntersuchungen ist es ohne weitere Automatisierung nicht mehr möglich, die Reaktionsansätze mit Hilfe von Wasserbädern zu temperieren. Des Weiteren kann innerhalb ein und desselben Wasserbades in der Regel nur eine Temperatur konstant gehalten werden, es ist nicht möglich, innerhalb kurzer Zeit bzw. innerhalb von definierten Zeitintervallen die Reaktionsansätze in einem Wasserbad mit verschiedenen Temperaturen, Temperaturintervallen oder Temperaturverläufen in Kontakt zu bringen.

Aus diesem Grunde wurde nach Alternativen gesucht, die zum einen eine homogene Temperierung von mehreren Reaktionsansätzen genauso ermöglichen wie den Wechsel von verschiedenen Temperaturen, das heißt die Abstimmung von spezifischen Temperatur- und Zeitintervallen. Realisiert wurde dieses Konzept mit Hilfe von Thermocyclern.

Thermocycler sind periodisch heizende Geräte, die in unterschiedlichen Ausführungen bereitgestellt werden, beispielsweise können die Geräte mit Hilfe von Flüssigkeiten heizen und kühlen, oder sie können sich über einen elektrischen Widerstand aufheizen und mit Halbleitern (Peltier) gekühlt werden. In der Regel besitzen die Thermocycler einen Metallblock, in den Aussparungen bzw. Löcher eingebracht sind, in denen der Reaktionsansatz, beispielsweise ein Tube, positioniert werden kann.

Sowohl die PCR als auch die Sequenzierung von Nukleinsäure- bzw. Aminosäurestrukturen sind junge, aber bereits etablierte Verfahren, deren weitere Entwicklung immer genauere und effektiv arbeitende Thermocycler bedingt. Diese Verfahren haben inzwischen - auch durch die weitere Verbesserung der Leistungsfähigkeit der Thermocycler - einen festen Stellenwert auch als diagnostische Methode sowohl in der Humanmedizin, Veterinärmedizin, Ernährungsindustrie, Biotechnologie als auch Pharmaforschung erreicht.

Bei der PCR-Reaktion ist das Verfahren im Wesentlichen über die Primersequenz und ein PCR-Protokoll mit spezifischen Temperatur-/Zeitintervallen definiert. Üblicherweise werden PCR-Verfahren in einer solchen Weise entwickelt, dass geringfügige Abweichungen der einzelnen Schritte der Istwerte des Temperaturprogramms des Thermocyclers von den Sollwerten einen möglichst geringen Einfluss auf das Ergebnis der PCR-Reaktion ausüben. Die dabei erzielte Robustheit der Reaktion ist für jedes PCR-Verfahren unterschiedlich. Bei jedem Verfahren wird aber bei einer vom jeweiligen Verfahren abhängigen Abweichung von den Sollwerten die Nachweisgrenze, also die Empfindlichkeit oder die Selektivität, beeinträchtigt. Der Nachweis ist also abhängig von der korrekten Temperatursteuerung des verwendeten Thermocyclers. Zur Vermeidung von falsch positiven bzw. falsch negativen Ergebnissen ist eine Kontrolle der korrekten Temperatursteuerung der verwendeten Geräte unerlässlich.

Insbesondere bei der Verwendung von Thermocyclern bei der PCR sollten Negativ- und eventuell auch Positivkontrollen mitlaufen. Trotz der Uniformität des Metallblockes werden selten eine homogene Temperaturverteilung und somit identische Ergebnisse bei identischen Reaktionsansätzen erreicht. Theoretisch sollte ein Metallblock überall die gleiche Temperatur aufweisen. In der Praxis kann die Temperatur von einem Reaktionsplatz zum anderen jedoch spürbar differieren, dementsprechend schwanken auch die Cyclerparameter und infolgedessen auch die erzielten Ergebnisse. Weiterhin variieren auch die Temperatur- und Zeitintervalle eines Metallblocks vor allem bei längerer Verwendung des Thermocyclers, das heißt, es ist nicht mehr gewährleistet, dass die angegebenen Gerätespezifischen Temperaturen korrekt in den vorgegebenen Zeitintervallen erreicht werden.

Insbesondere beim Einsatz von so genannten Multiblocksystemen für Großanwender mit hohem Durchsatz kann eine homogene, gleichbleibende Temperaturverteilung (i) innerhalb der Heizblocks und (ii) innerhalb der Zeitintervalle als absolut erreichte Temperatur an einem definierten Ort vor allem bei längerem Gebrauch (z.B. Jahre) des Gerätes in der Regel nicht gewährleistet werden.

Derzeit besitzen beinahe alle Geräte einen standardisierten Block zur Aufnahme von PCR-Reaktionsgefäßen. Die Temperatursteuerung der Geräte ist nicht standardisiert und unterscheidet sich je nach Hersteller des Thermocyclers und Modells erheblich. Bedingt durch die Verwendung von Peltier-Elementen zeigen alle Geräte außerdem eine nutzungsabhängig nachlassende Leistung (Performance) in der präzisen Temperierung, eine Folge der Alterung der Peltier-Elemente. Die korrekte Einhaltung der Temperatur ist aber für die Spezifität und die Sensitivität eines PCR-Nachweises entscheidend.

Eine Kontrolle der Temperaturverteilung auf dem Heizblock und die Kontrolle der Temperatursteuerung findet zum Beispiel durch die Messung mit physikalisch-technischen Messverfahren statt. Hierzu ist es erforderlich, den jeweiligen Thermocycler einzuschicken, das heißt, es ist keine Überprüfung während des laufenden Betriebes im Labor im Rahmen des Qualitätsmanagements möglich. Die räumliche Gestaltung der Messsonden erzeugt nachteilhafterweise von Realität abweichende thermische Verhältnisse im Thermocycler, was im Wesentlichen bedeutet, dass die Ergebnisse von der Laborrealität abweichen.

Ein anderes Verfahren im Stand der Technik ist die Messung mit nicht standardisierten PCR-Verfahren innerhalb der Labore. Nachteilig ist jedoch hierbei, dass alle für die Routine benutzten PCR-Verfahren mehr oder weniger sehr robuste Systeme sind, das heißt, Veränderungen können erst dann detektiert werden, wenn dieses bereits sehr robuste System gestört wird. Insbesondere ist keine Aussage darüber möglich, wie die Robustheit des verwendeten Verfahrens zu allen anderen PCR-Verfahren des Labors steht. Daraus kann geschlussfolgert werden, dass es eigentlich gegen alle anderen Verfahren des Labors regelmäßig überprüft werden müsste, was jedoch praktisch unmöglich ist. Es sind außerdem keine getrennten Aussagen über die Probleme bei der Denaturierung und/oder des Annealings möglich.

Der Erfindung liegt demgemäß die Aufgabe zugrunde, ein Verfahren bereitzustellen, das die genannten Nachteile vermeidet, die sich insbesondere aus der Messung mit physikalisch-technischen Messverfahren oder mit der Messung mit nicht standardisierten PCR-Verfahren innerhalb der Labore ergeben, wobei das Verfahren so ausgestaltet sein soll, dass es eine einfache, sichere und effiziente Validierung und Referenzierung von Thermocyclern erlaubt.

Die Erfindung löst diese technische Aufgabe durch Bereitstellung eines Verfahrens zur Validierung oder Referenzierung von Thermocyclern, wobei mindestens ein PCR-Reaktionsansatz in dem Thermocycler positioniert oder mit diesem wirkverbunden ist und mindestens eine Prüfung der Annealingtemperatur und der Denaturierungstemperatur vorgenommen wird.

Die Erfindung betrifft also die überraschende Lehre, dass durch die Überprüfung von zwei Temperaturen, nämlich der Annealingtemperatur und der Denaturierungstemperatur, eine Referenzierung von Thermocyclern ohne die bekannten Nachteile möglich ist. Durch die Bestimmung von zwei Temperaturen ist es möglich, Thermocycler ohne die Messung mit physikalisch-technischen Messverfahren, wie beispielsweise Messsonden, zu validieren. Des Weiteren sind getrennte Aussagen über die Probleme bei der Denaturierung und/oder dem Annealing möglich, wobei das erfindungsgemäße Verfahren vorteilhafterweise gegen alle anderen Verfahren im Labor einfach und sicher regelmäßig überprüft werden kann.

Das erfindungsgemäße Verfahren umfasst demgemäß zwei PCR-Reaktionen, die insbesondere in einem Gefäß ablaufen, wobei das erste PCR-Verfahren die Annealingphase und das zweite PCR-Verfahren die Denaturierungsphase überprüft. Die Verfahren benutzen insbesondere einen unterschiedlichen Primer und bilden Produkte unterschiedlicher Länge und/oder Sequenzabfolge. Das Verfahren ist so ausgebildet, dass die PCR-Verfahren eine besonders hohe Temperaturempfindlichkeit besitzen.

Bei der erfindungsgemäßen Validierung des Thermocyclers ist es insbesondere möglich, dass eine bestimmte Anzahl von Reaktionsansätzen über die Fläche des Heizblocks eines Thermocyclers - nach einem statistischen Zufallsmuster, willkürlich und/oder geordnet - verteilt wird. Die Positionierung richtet sich hierbei nach dem zu untersuchenden Thermocycler. Ein Thermocycler kann beispielsweise 5, 10, 24, 96, 384 oder mehr Kavitäten aufweisen.

Eine Polymerase Chain Reaction im Sinne der Erfindung ist jede Methode zur Vermehrung von DNA. Sie ist bevorzugt eine thermozyklische Reaktion, wobei in jedem Zyklus theoretisch die zu Zyklusbeginn bereitgestellte DNA Menge verdoppelt wird. Unter Annealing versteht man im Sinne der Erfindung die Anlagerung des Primers an das DNA-Template. Nach dem Aufschmelzen der gesamten DNA wird das Reaktionsgemisch auf eine sehr genau definierte Temperatur abgekühlt. Bei dieser sogenannten Annealingtemperatur kommt es zur spezifischen Bindung des Primers an sein Template. Allerdings auch zur Renaturierung des ursprünglichen Doppelstranges und zur unspezifischen Anlagerung. Die Renaturierung wird z.B. durch den hohen Primerüberschuß verhindert. Erst nach Bindung der Primer renaturiert der Rest des Templates ohne den Primer zu verdrängen. Die unspezifische Anlagerung wird dadurch vermieden, dass die Annealingtemperatur nur ganz schwach (ca. 2°) unterhalb der Schmelztemperatur liegt. Naturgemäß ist die spezifische Bindung stabiler. Das nutzt man aus: An ihren stabilen Bindungsstellen binden die Primer gerade noch stabil genug, an den unspezifischen Bindungsstellen kommt es dagegen zu einem fortlaufenden Abschmelzen. In kritischen Fällen kann man auch die Annealingtemperatur höher als den Schmelzpunkt wählen. Das vermindert die Ausbeute, erhöht aber die Spezifität. Weil die Basenfolge der Primer bekannt ist, kann man stets seine Schmelztemperatur und damit auch die Annealingtemperatur berechnen. Unter Einbeziehung aller möglichen Faktoren - hauptsächlich der Purin-Pyrimidin-Verteilung - ist die Schmelztemperaturen mit einer Genauigkeit bis auf die Nachkommastelle bestimmbar. In der Regel reicht die Anwendung der Wallace-Regel aus. Dazu zählt man getrennt Adenosine und Thymidine bzw. Guanosine und Cytidine aus. Der AT-Wert wird mit 2, der GC-Wert mit 4 multipliziert. Die Addition ergibt die Schmelztemperatur in °C. Typische Primer ergeben Werte zwischen 55 und 65°C. Die Schmelztemperatur sollte für beide Primer möglichst gleich sein. Unterscheiden sich die beiden Temperaturen, so muß bei der niedrigeren Temperatur gearbeitet werden. Das führt beim Gegenprimer zur Erhöhung der Unspezifität. Im Lauf der Reaktion nimmt die Konzentration an Primer stark ab. Um auch in diesem Bereich eine optimale Bindung zu erreichen, kann man die Annealingzeit variieren. Liegt das DNA-Template nur in sehr geringer Konzentration vor, oder ist das Reaktiongemisch nicht sauber, z.B. durch starke Fremdionen verschmutzt, so kann eine Verlängerung der Annealingzeit hilfreich sein, damit der Primer sein Template findet.

Unter Denaturierung versteht man im Sinne der Erfindung die Strangtrennung. Der Mechanismen der Strangtrennung ist abhängig von der Architektur und Stabilität der Doppelhelix. Die Stapelkräfte beruhen auf Wechselwirkungen zwischen den Π-Elektronensystemen der quasi aromatischen Basen. Der Effekt wird auch als charge transfer Komplex bezeichnet. Ab einer Länge von etwa 30 Nukleotiden ist die Stabilität der Doppelhelix nicht mehr von der Länge des Polymers abhängig. Ein streng kooperatives Verhalten ist nachweisbar. Das gilt für eine statistische Verteilung der Nukleotide. GC-reiche Abschnitte zeigen dagegen eine erhöhte Stabilität gegenüber AT-reichen Abschnitten. Andere Faktoren, die die Stabilität beeinflussen, können die Überstruktur und assoziierte Proteine sein. Bei Oligonukleotiden (bis circa 30 Basen) und an den Ende liegen besondere Verhältnisse vor. Hier ist die Stabilität der Helix noch von der Länge der DNA, bzw. vom Abstand zum Ende abhängig; die Enden sind am wenigsten stabil. Das endständige Nukleotid kann nur in eine Richtung Stapelkräfte ausbilden. Folglich ist es auch bei niedrigen Temperaturen fast immer frei. Das zweite Nukleotid ist gleichfalls relativ frei, wenn das erste Nukleotid keine Stapelkräfte ausbildet. So sind Stabilitätsgradienten beobachtbar. Erst ab Nukleotid vier kann der Effekt vernachlässigt werden. Das wiederum bedeutet, dass ein Octa-oligonukleotid (8-mer) das erste Oligomer mit einer messbaren Stabilität ist, vorausgesetzt die Sequenz ist selbstkomplementär. Bereits bei Raumtemperatur ist diese Doppelhelix nicht mehr stabil.

In einer bevorzugten Anwendungsform der Erfindung erfolgt die Validierung im Zusammenhang mit einer Real-Time PCR. In der Real-Time PCR wird die Entstehung der PCR Produkte während der Amplifikation gemessen. Das Verfahren beruht auf Fluoreszenz-Änderungen, die durch die transparenten Gefäßwandungen oder den Deckel gemessen werden. Fluoreszenz ist die Eigenschaft von Substanzen, aufgenommene Energie als Lichtquant niedrigerer Energie wieder abzugeben. In den letzten Jahren sind Real-Time PCR Geräte auf den Markt gekommen, mit denen verschiedene Real-Time PCR Verfahren angewendet werden können. Die einfachste Real-Time PCR Methode beruht auf der Zugabe von SYBR Green, einem Farbstoff, der bei Anlagerung an die doppelsträngigen PCR Produkte seine Fluoreszenz erhöht. Entgegen der klassischen Methode der Detektion im Agarose-Gel geht die zusätzliche Information über die Länge des Produktes verloren; die Methode ist daher nicht geeignet, das PCR Experiment zu verifizieren. Durch eine anschließende Schmelzanalyse kann jedoch eine grobe Einschätzung vorgenommen werden; zumindest Primer-Dimere als häufig präsente Nebenprodukte können in der Schmelzanalyse erkannt werden. Alle anderen Methoden bedienen sich fluoreszent markierter Primer oder Sonden.

Die älteste und wahrscheinlich am weitesten verbreitete Methode ist der 5-Nuklease Assay, besser bekannt als TaqMan Assay. Hierbei wird eine doppelt markierte Oligonukleotid-Sonde verwendet, die einen Reporter-Fluoreszenzfarbstoff und einen Quencher trägt. Der Quencher löscht oder mindert die Fluoreszenz des Reporters durch Kollision der Sonden-Enden, oder durch einen Fluoreszenz-Resonanz-Energietransfer (FRET). Die Sonde bindet an das entstehende PCR-Produkt und wird von der Polymerase hydrolysiert, so dass Reporter und Quencher getrennt werden, wodurch die Fluoreszenz zunimmt. Dieses Signal ist demnach von der Existenz der Bindungsstelle abhängig und verifiziert die PCR-Reaktion.

Bevorzugt ist auch eine Touchdown PCR, bei der die Spezifität der Primerbindung durch zyklusweise Annäherung der Annealingtemperatur an Tm erhöht wird. Bei der Touchdown PCR wird die Annealingtemperatur der zu erwartenden Schmelztemperatur des Primers (TM) zyklusweise angenähert und über diese hinaus abgesenkt. Wenn die Annealingtemperatur über der zu erwartenden Tm gewählt wird, binden die Primer ausschließlich und damit hochspezifisch an die DNA. So werden Primerdimere und Artefakte verringert, und es vermehrt sich vorzugsweise das gewünschte Amplikon.

Bevorzugt ist weiterhin eine Prolongations PCR, bei der durch Verlängerung der Elongationphase der allelic-dropout-effect vermieden werden soll. Die Prolongations PCR ist eine Methode, bei der der letzte Schritt eines jeden Zyklus velängert wird. Dies soll den "allelic-drop-outeffect" verhindern. Man will den Abbruch der Synthese vor Erreichen des Endproduktes verhindern. Verlängert man die Elongationsphase proportional zur Abnahme an aktiven Enzymen, Template und dNTP's, so wird der Endpunkt stets erreicht, und alle Kopien des Gens haben dieselbe Chance amplifiziert zu werden. Die Synthese gewinnt an Effizienz und auch längere DNA Fragmente werden vervollständigt. Diese Methode wird vornehmlich bei heterogenen DNA Gemischen verwendet. Sie wird vor allem genutzt, wenn zwei Polymerasen gleichzeitig eingesetzt werden wie z.B. bei dem EXPAND-PCR-System von ROCHE. Dabei sorgt eine Polymerase für eine schnelle Synthese und die andere für die Qualitätskontrolle mittels "proofreading". Allelicdrop-out-effect: In einem heterogenen DNA Gemisch wie z.B. bei phorensischen Bestimmungen, sind bestimmte Allele unterschiedlich häufig vertreten. Da die PCR exponentiell amplifiziert, kann diese Ungleichverteilung so sehr verstärkt werden, dass das geringer konzentrierte Allel im Verhältnis so gering vertreten ist, dass es nicht mehr zu detektieren ist.

Bevorzugt ist weiterhin auch eine hot start PCR, beid er die Polymerisation erst bei Erreichen einer Mindesttemperatur beginnt. Die hot start oder auch Heißstart PCR unterscheidet sich von dem allgemeinen Verfahren nur im ersten Schritt. Die Polymerasekettenreaktion wird in diesem Fall erst gestartet, wenn das Reaktionsgemisch die gewünschte Höchsttemperatur erreicht hat. Damit erreicht man, dass die Polymerisation erst beginnt, wenn die Primer spezifisch an die DNA Sequenz gebunden haben. Man erhält weniger Artefakte. Dies kann durch unterschiedliche Verfahren erreicht werden. Die ursprüngliche Variation war die Zugabe der Polymerase erst nach Erreichen der Schmelztemperatur der DNA. Dieses Verfahren birgt einige Nachteile. Weil die Polymerase per Hand nacheinander zu vielen Proben pipettiert wird, werden die Reaktionen nicht mehr zeitgleich gestartet. Außerdem kann es durch das Öffnen des Reaktionsgefäßes zu Verunreinigungen kommen. Eine weitere Methode ist das Trennen der Reaktionskomponenten durch eine Wachsschicht. Dabei werden zuerst die Templates, Puffer, dNTPs, Primer und Supplime in das Reaktionsgefäß gegeben. Danach gibt man ein Wachskügelchen definierter Zusammensetzung (im Handel erhältlich) dazu. Beim ersten Erhitzen schmilzt das Wachs und versiegelt die unvollständige Reaktionsmixtur. Darauf gibt man die Polymerase. Schmilzt das Wachs beim Erhitzen und steigt auf, mischen sich die wässrigen Phasen. Der Vorteil dieser Methode ist, daß alle Reaktionsansätze gleichzeitig gestartet werden. Durch die definierten chemischen Eigenschaften des Wachses, sind die Experimente gut reproduzierbar. Als Drittes besteht die Möglichkeit des verzögerten Startens der Reaktion durch Verwendung von Antikörpern. In diesem Fall wird eine DNA Polymerase verwendet, die an Antikörper gebunden ist. Die Antikörper blockieren die Polymeraseaktivität bei normaler Umgebungstemperatur. Da die Antikörper nicht thermostabil sind, denaturieren und dissoziieren sie beim ersten Erhitzen. Jetzt kann die Polymerase mit ihrer Arbeit beginnen. Dies ist eine spezifische Methode, da die Polymerase erst aktiv ist, wenn die Temperatur so hoch ist, daß die Primer durch die höhere Temperatur spezifisch an die DNA binden. Die Vorteile der hot start Methode sind: Es reichern sich weniger oder keine Artefakte an; die Polymerase-Reaktion beginnt erst, wenn die DNA vollständig aufgeschmolzen ist; die Polymerisation beginnt erst, wenn die Primer auf Grund der Temperatur spezifisch an die DNA binden und die Primerkonzentration wird nicht durch Primerdimerisierung verringert.

Bei der ebenfalls bevorzugten nested PCR wird nach erfolgreicher erster Amplifikation das erste PCR Produkt als Template für eine zweite PCR verwendet. Die nested-PCR ist eine Methode um die Spezifität der PCR weiter zu erhöhen. Hierbei wird in einem ersten Schritt die PCR mit einem Template über wenige Zyklen durchgeführt. Anschließend werden in einer zweiten Runde neue Primer eingesetzt. Die neuen Primer liegen weiter innen als die ersten, so dass in diesem zweiten Schritt nur noch die spezifischen DNA Abschnitte des ersten Schritts amplifiziert werden. Es kann auch nur ein weiter innenliegender Primer eingesetzt werden. Dies erhöht die Empfindlichkeit und die Effizienz so, dass sehr sorgfältig und kontaminationsfrei gearbeitet werden muss, da Verunreinigungen ungewünschte Auswirkungen haben können.

Die ebenfalls bevorzugte RT-PCR dient zum Nachweis von RNA oder einer zur RNA komplemantären doppelsträngigen DNA. Mit dieser Methode kann man Untersuchungen von Expressionsmustern auf mRNA Ebene durchführen. Außerdem kann durch die RT-PCR hoch sensitiv virale RNA nachgewiesen werden. Die RT-PCR ist in zwei Schritte unterteilt. Im ersten Schritt wird die RNA durch Reverse Transkription in cDNA (c steht für komplementäre) umgeschrieben. Dies ist nötig, da viele Polymerasen nicht in der Lage sind RNA als Template zu verwenden. Im zweiten Schritt schließt sich eine normale PCR an, in der die im ersten Schritt erzeugte cDNA als Template verwendet wird. Es gibt die Möglichkeit, diese beiden Schritte räumlich und/oder zeitlich voneinander zu trennen. Die herkömmliche Methode ist es, erst Reverse Transkriptase in das Reaktionsgemisch zu geben, und später dieses als Template in den PCR Ansatz einzusetzen. Eine andere Methode ist es, Tth-Polymerase (aus Thermus thermophilus) zu verwenden. Tth- Polymerase kann sowohl als Reverse Transkriptase als auch als Polymerase arbeiten. Die Regulation übernehmen bestimmte Kofaktoren: In Anwesenheit von Mangan-Ionen zeigt die Tth-Polymerase eine Reverse Transkriptase Aktivität, und in Anwesenheit von Magnesium-Ionen eine DNA Polymerase Aktivität. So kann man durch Zugabe der richtigen Kofaktoren die gesamte RT-PCR in einem Ansatz durchführen.

In einer weiteren bevorzugten Ausführungsform der Erfindung wird die Annealing- und die Denaturierungstemperatur in mindestens zwei Reaktionsansätzen überprüft. Vorteilhafterweise wird in einem Reaktionsansatz die Annealingtemperatur und in einem zweiten Reaktionsansatz die Denaturierungstemperatur überprüft. Mit Vorteil interagieren die Komponenten, die zur Bestimmung in dem Reaktionsansatz verwendet werden, so nicht miteinander.

In einer weiteren bevorzugten Ausführungsform der Erfindung wird zur Überprüfung der Temperatur ein DNA-Template eingesetzt, wobei das DNA-Template durch einen hohen T_{M}-Wert mindestens eines Abschnittes temperatursensitiv ist. Vorteilhafterweise ist es durch die Auswahl des den hohen T_{M}-Wert aufweisenden Abschnittes des DNA-Templates möglich, die Denaturierungstemperatur zu überprüfen und zu referenzieren.

In einer weiteren bevorzugten Ausführungsform der Erfindung wird zur Überprüfung der Temperatur, insbesondere der Annealingtemperatur, mindestens ein Primer eingesetzt, wobei der Primer durch ein Mismatch dergestalt sensitiv ist, dass die Annealingtemperatur bestimmt werden kann. Die Temperaturempfindlichkeit der PCR wird vorteilhafterweise über mindestens eine Fehlpaarung, insbesondere am 3'-Ende mindestens eines Primers erzielt, wodurch eine Überprüfung der Annealingphase der PCR-Reaktion möglich ist. Durch die eine oder durch mehrere Fehlpaarungen am 3'-Ende eines Primers wird mit Vorteil eine Herabsetzung der Effektivität erreicht, wodurch die PCR-Reaktion im Gegensatz zu den üblichen Reaktionen nicht sehr stabil und robust ist und somit gegenüber Temperaturabweichungen sehr sensitiv wird.

Der mindestens eine Fehlpaarung am 3'-Ende aufweisende Primer kann insbesondere in einem Mastermix verwendet werden. Bevorzugt enthält der Mastermix alle Bestandteile, die für die Durchführung einer PCR benötigt werden; bevorzugt PCR-Puffer, MgCl₂, dNTP's und/oder Taq-Polymerase. Für die Durchführung einer Real-Time-Detektion enthält der Mastermix weiterhin die dem Fachmann bekannten erforderlichen Sonden.

In einer weiteren bevorzugten Ausführungsform der Erfindung umfasst der Primer insbesondere bei der Überprüfung der Annealingtemperatur folgende Sequenzen: Primer 1  GATACAGAAACATCGGTTGGC und

Primer 2  GTGTAACTTGATGCCATCAGG.

In einer weiteren bevorzugten Ausführungsform der Erfindung wird die Annealingtemperatur in dem PCR-Ansatz mit einer Target-DNA mit der Sequenz 
GATACAGAAACATCGGTTGGTGATCGATCGAGGTTTTTAAAAACCCCCTCTAGCTAG
CTAGCTAGCGATTGCTTCACCAAGAAGAGCTCCAACAGCCTGATGGCATCAAGTTAC
AC
 überprüft.

In einer besonders bevorzugten Ausführungsform der Erfindung wird die Denaturierungstemperatur in dem PCR-Reaktionsansatz überprüft, indem ein hochschmelzendes PCR-Template eingesetzt wird, wobei die T_{M} mindestens eines Abschnittes in dem Template größer als 90 °C ist.

Dem Fachmann sind verschiedene Möglichkeiten bekannt, den T_{M}-Wert des Abschnittes in einem Template zu erhöhen, insbesondere ist es möglich, den jeweiligen Abschnitt besonders GC-Reich zu wählen.

In einer weiteren bevorzugten Ausführungsform weist das Template bevorzugt zur Bestimmung der Denaturierungstemperatur folgende Sequenz auf:

TCACGAGACACCCTGCCGCCCTCTTCTTTCCCGCCATTCCCTCCCTACCCTTCAAAA
TCTAGAAACCTTTTTTTTCCTCCCGATACGCCCCTCCATCTCCCTCGCCGTTCATG
TCCGTGGCTGGCTGCCCTCCGTGGGAGCAGGCGGCCGCACTCGTTCCCCGCCGCAGC
CGATCCAACAATGGACAACAACCCCAACATCAACGAGTGCATCCCCTACAACTGCCT
GAGCAACCCCGAGGTGGAAGGTGCTGGCGGCGAGCGCATCGAGACCGGCTACACCC
CCAAATCGACATCAGCCTGAGCCTGACCCAGTTCCTGCTGAGCGAGTTCGTGCCCGG
CGCCGGCTTCGTGCTGGGCCTGGTGGACATCATCTGGGGCATCTTCGGC.

In einer weiteren bevorzugten Ausführungsform der Erfindung wird insbesondere die Denaturierungstemperatur in dem PCR-Reaktionsansatz durch die Verwendung von Primern mit folgenden Sequenzen überprüft: Primer 1  TCACGAGACACCCTGCCG und

Primer 2  GCCGAAGATGCCCCAGAT.

In einer besonders bevorzugten Ausführungsform der Erfindung wird eine definierte Menge an zwei verschiedenen Target-DNAs in einem PCR-Reaktionsansatz eingesetzt. Es ist beispielsweise möglich, dass die Reaktionsansätze beispielsweise 10.000, 1.000 oder 100 Kopien der Ausgangs- oder Target-DNA enthalten. Durch Routineversuche kann der Test vorteilhafterweise so eingestellt werden, dass bei einem korrekt arbeitenden Temperaturcycler aller Reaktionen mit 10.000 und 1.000 Kopien ausreichend PCR-Produkte generieren, die beispielsweise in einer nachfolgenden gelelektrophoretischen Auswertung deutlich als Bande zu detektieren sind. Selbstverständlich ist die Auswahl der definierten Mengen in nahezu jedem Bereich möglich. Es können beispielsweise auch 20, 200, 2.000, 20.000 und 200.000 oder 50, 500 und 5.000 Kopien verwendet werden, die nach Durchführung der Überprüfung der Annealingtemperatur und der Denaturierungstemperatur verglichen werden können. Bevorzugt ist es, dass 2 oder 3, aber auch 4, 5, 6 oder auch mehr verschiedene definierten Mengen von beiden der jeweiligen Target-DNAs eingesetzt werden.

Bevorzugt ist weiterhin, dass die Überprüfung der Annealingtemperatur und die Überprüfung der Denaturierungstemperatur als Multiplex-Reaktion durchgeführt werden. Das heißt, vorteilhafterweise werden alle zu den jeweiligen Reaktionen passenden Primer zeitgleich eingesetzt.

In einer weiteren bevorzugten Ausführungsform der Erfindung wird die Überprüfung der Annealingtemperatur und der Detanurierungstemperatur mittels getrennter Auswertung von PCR-Produkten unterschiedlicher Größe und/oder Sequenz durchgeführt. Es ist insbesondere möglich, bei PCR-Produkten unterschiedlicher Größe die Produkte im Gel zu untersuchen. Sofern Real-Time-Verfahren eingesetzt werden, ist es vorteilhafterweise möglich, die unterschiedlichen Sequenzen zu untersuchen. Bei der klassischen PCR ist es bevorzugt, aus einer Multiplex-PCR mindestens beide Produkte in eine Geltasche zu bringen und bei der Real-Time-PCR ist es bevorzugt, ein Multiplex-PCR mit Real-Time-Sonden durchzuführen.

In einer besonders bevorzugten Ausführungsform der Erfindung werden 12 bis 17 PCR-Reaktionsansätze verwendet. Die Anzahl der PCR-Reaktionsansätze richtet sich selbstverständlich insbesondere nach dem so genannten Microtiterplattenlayout auf dem PCR-Block.

Bei einem standardisierten Microtiterplattenlayout sind 12 x 8 Aussparungen bzw. Löcher in Thermoblöcken bzw. dem PCR-Block vorhanden; diese 96-well-Anordnungen werden in vorteilhafter Weise in verschiedensten Laborroutinen in Klinik und Grundlagenforschung verwendet. Es können jedoch auch Thermoblöcke mit 192, 384 oder mehr Aussparungen, Löchern oder Bohrungen verwandt werden. Bei einem 96-well-Layout werden beispielsweise Proben - gemäß der standardisierten Kennzeichnung des Microtitaplattenlayouts (waagerecht 1-12, senkrecht A bis H) - an den Positionen A1, B2, C3, A10, B11, C12 sowie F1, G2, H3 sowie F10, G11 und H12 fixiert bzw. so positioniert, dass sie mit diesen Orten auf dem Metallblock wirkverbunden sind, wobei an den jeweiligen vier äußeren Ecken jeweils drei Proben in einer Linie angebracht sind. Weiterhin werden insbesondere drei Proben auf den Positionen D5, D6 und D7 positioniert. Es kann beispielsweise in solch einem Fall vorgesehen sein, dass in den Reaktionsansätzen A1, F1, D5, A10 und F10 jeweils die gleiche Anzahl von Kopien, beispielsweise 10.000 Kopien, eingebracht ist, und in den Positionen B2, B11, G2, G11 und D7 jeweils 1.000 Kopien und in den restlichen Positionierungen jeweils 100 Kopien eingebracht sind.

Durch die Offenbarung dieser Positionierung bei einem - mit einem 96-well-Microtiterplattenlayout gestalteten - PCR-Block ist es dem Fachmann möglich, für alle anderen beliebigen Layouts Gruppierungen durch Routineversuche oder einfaches Überlegen zu wählen.

In einer weiteren besonders bevorzugten Ausführungsform der Erfindung ist die Anordnung der PCR-Reaktionsansätze im Wesentlichen der Anordnung der Peltier-Elemente und/oder der Temperatursonden des Thermocyclers angepasst.

Vorteilhafterweise werden die PCR-Reaktionsansätze so positioniert, dass sie optimal der Anordnung der Heizungs- und Kühlelemente angepasst sind, um so eine möglichst objektive Messung der Annealing- und der Denaturierungstemperatur zu ermöglichen, wobei die Messung der einzelnen Reaktionsansätze, die auf dem PCR-Block angeordnet sind, Hinweise auf sämtliche mögliche Reaktionsorte auf dem PCR-Block geben kann.

In einer weiteren besonders bevorzugten Ausführungsform der Erfindung wird in dem PCR-Reaktionsansatz folgende Reaktion durchgeführt,
- Anfangsdenaturierung: 1 min, 95 °C in 20 Zyklen mit folgenden Phasen:
- Denaturierung: 10 sec, 95 °C, Annealing: 20 sec, 63 °C, Elongation: 20 °C, 72 °C und terminale Elongation: 5 sec, 72 °C.

Die Erfindung betrifft auch einen Referenzierungskit, wobei dieser mindestens einen Primer mit mindestens einer Fehlpaarung am 3'-Ende und ein hochschmelzendes PCR-Template umfasst, bei dem die T_{M} mindestens eines Abschnittes in dem Template größer als 90 °C ist. Der Referenzierungskit kann beispielsweise als Reaktionsansatz für die Validierung bzw. Referenzierung von PCR-Blöcken verwendet werden. Der Kit kann eine Information, beispielsweise einen Beipackzettel, umfassen, der Informationen über die Verwendung der einzelnen Bestandteile des Kits enthält, bevorzugt zur Validierung und Referenzierung von Thermocyclern.

Bevorzugt ist es, dass mindestens eine Target-DNA mit einer Wand des Reaktionsansatzes, zum Beispiel einem Tube, assoziiert ist. Besonders bevorzugt ist es, dass beide Target-DNAs und insbesondere auch die jeweiligen Primer mit der Wand des PCR-Reaktionsansatzes assoziiert sind. Der Kit kann beispielsweise mindestens einen, 10, 20 oder mehrere PCR-Reaktionsansätze umfassen, bei denen an der Innenwand mindestens eine, bevorzugt beide oder mehrere Target-DNAs assoziiert sind.

Die Erfindung betrifft auch die Verwendung des Kits bzw. die Verwendung des erfindungsgemäßen Verfahrens zur Durchführung der Validierung oder Referenzierung von Thermocyclern.

Das erfindungsgemäße Verfahren, der Kit und die Verwendung weisen mehrere Vorteile gegenüber dem Stand der Technik auf:

Verfahren und Kit sind jederzeit im Labor, das heißt unter realen Bedingungen, einsetzbar. Damit wird die regelmäßige Überprüfung von Thermocyclern genauso ermöglicht wie der sofortige Einsatz, wenn ungelöste Probleme während der PCR auftreten.

Ein besonderer Vorteil besteht in der Erfassung von Verschiebungen in der Temperatursteuerung sowohl in zu niedrigeren Temperaturen als zu höheren Temperaturen als eingestellt oder laut Protokoll vorgeschrieben. Durch die spezielle Konstruktion der Thermocycler treten Temperaturungenauigkeiten und Temperaturabweichung praktisch immer in einer Richtung auf.

Mit dem vorgestellten Verfahren wird mit der Reaktion zur Überprüfung zur Denaturierungstemperatur eine Verschiebung hin zu geringeren Temperaturen als eingestellt erfasst und mit der Reaktion zur Überprüfung der Annealingtemperatur eine Verschiebung zu höheren Temperaturen als eingestellt ebenfalls erfasst.

Mit diesem System werden also vorteilhafterweise Verschiebungen in beide Richtungen und damit besonders bevorzugt alle möglichen Abweichungen erfasst. Dieses Verfahren kann mit Vorteil als Laborroutine und demgemäß als Routineüberprüfung in einem Laboratorium eingesetzt werden, das eine ständige Kontrolle seiner Prüfmittel benötigt, wie beispielsweise akkreditierte Laboratorien oder ISO 9001 und andere.

Im Folgenden soll die Erfindung anhand eines Beispiels zumindest teilweise in den Figuren näher erläutert werden, ohne auf dieses Beispiel beschränkt zu sein.

### Beispiel:

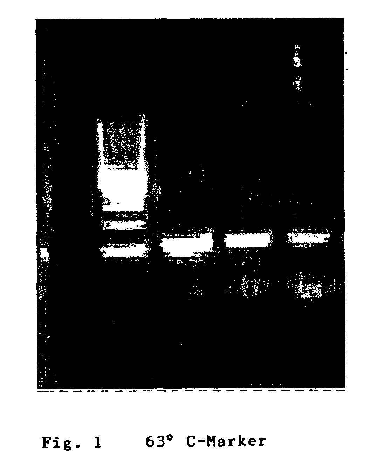

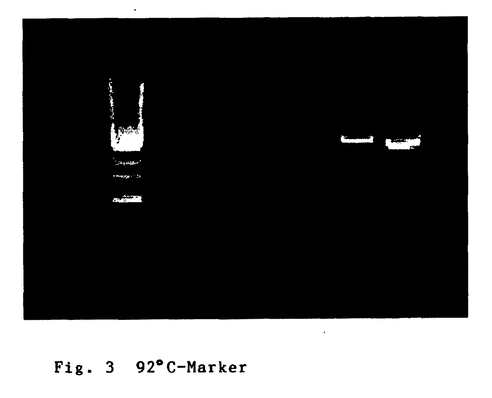

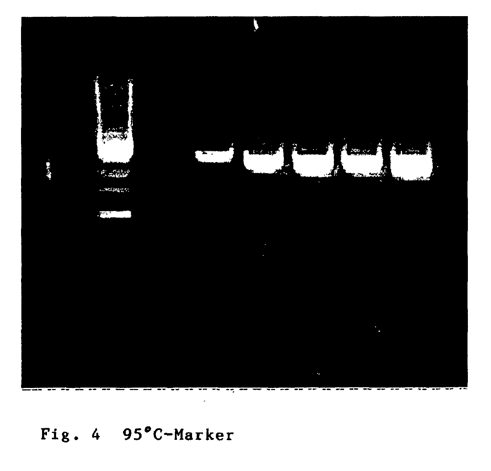

Nach Beendigung der PCR werden die Reaktionsprodukte auf ein Gel aufgetragen, aufgetrennt und mittels einer üblichen Färbereagenz sichtbar gemacht. Die Detektion erfolgt visuell oder mit einer Kamera (siehe Fig. 1 bis 4) .

In einer bevorzugten Variante werden 5 µl der Reaktion über eine Agarosegelelektrophorese aufgetrennt und anschließend das Produkt über eine Färbung sichtbar gemacht.

Falls die Annealingtemperatur in allen Blockpositionen korrekt eingehalten wurde, dann ist in allen Reaktionen mit a und b als Ausgangs-DNA eine deutliche Bande detektierbar. Die Reaktionen mit der Ausgangsmenge c ergeben nur eine schwache Bande.

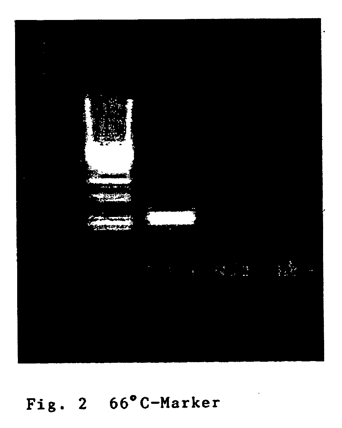

Im Falle der Nichteinhaltung der Annealingtemperatur verschwindet mehr als eine Bande. In den Figuren 1 und 2 ist dieses Verhalten dargestellt, wobei 63 °C die korrekte Temperatur und 66 °C eine zu hohe Annealingtemperatur ist.

Falls die Denaturierungstemperatur in allen Blockpositionen korrekt eingehalten wurde, dann ist in allen Reaktionen mit a und b als Ausgangs-DNA eine deutliche Bande detektierbar. Die Reaktionen mit der Ausgangsmenge c ergeben nur eine schwache Bande.

Im Falle der Nichteinhaltung der Denaturierungstemperatur verschwindet mehr als eine Bande (siehe Fig. 1 bis 4).

## Patentansprüche

1. Verfahren zur Validierung oder Referenzierung von Thermocyclern,
**dadurch gekennzeichnet, dass**
mindestens ein PCR-Reaktionsansatz in dem Thermocycler positioniert oder mit diesem wirkverbunden ist und mindestens eine Überprüfung der Annealingtemperatur und der Denaturierungstemperatur vorgenommen wird.

2. Verfahren nach Anspruch 1,
**dadurch gekennzeichnet, dass**
es in einer Real-Time-PCR durchgeführt wird.

3. Verfahren nach einem der vorhergehenden Ansprüche,
**dadurch gekennzeichnet, dass**
zu der Überprüfung der Annealingtemperaturen mindestens ein Primer eingesetzt wird, der durch mindestens einen Mismatch temperatursensitiv ist.

4. Verfahren nach einem der vorhergehenden Ansprüche,
**dadurch gekennzeichnet, dass**
der Primer als Mismatch eine Fehlpaarung am 3'-Ende aufweist.

5. Verfahren nach einem der vorhergehenden Ansprüche,
**dadurch gekennzeichnet, dass**
ein DNA-Template eingesetzt wird, das durch einen hohen T_{M}-Wert mindestens eines Abschnittes temperatursensitiv ist.

6. Verfahren nach einem der vorhergehenden Ansprüche,
**dadurch gekennzeichnet, dass**
die Denaturierungstemperatur in dem PCR-Reaktionsansatz überprüft wird, indem ein hochschmelzendes PCR-Template eingesetzt wird, wobei die T_{M} mindestens eines Abschnittes in dem Template größer als 90 °C ist.

7. Verfahren nach einem der vorhergehenden Ansprüche,
**dadurch gekennzeichnet, dass**
in mindestens einem PCR-Reaktionsansatz eine definierte Menge an zwei verschiedenen Target-DNAs eingesetzt wird.

8. Verfahren nach einem der vorhergehenden Ansprüche,
**dadurch gekennzeichnet, dass**
zwei oder drei verschiedene Mengen von beiden der jeweiligen Target-DNAs eingesetzt werden.

9. Verfahren nach einem der vorhergehenden Ansprüche,
**dadurch gekennzeichnet, dass**
die Überprüfung der Annealingtemperatur und die Überprüfung der Denaturierungstemperatur als Multiplexreaktion durchgeführt werden.

10. Verfahren nach einem der vorhergehenden Ansprüche,
**dadurch gekennzeichnet, dass**
die Überprüfung der Annealingtemperatur und der Denaturierungstemperatur mittels getrennter Auswertung von PCR-Produkten unterschiedlicher Größe und/oder Sequenz durchgeführt wird.
